(19) Europäisches Patentamt / European Patent Office / Office européen des brevets

(11) **EP 3 017 816 B1**

(12) **EUROPEAN PATENT SPECIFICATION**

(45) Date of publication and mention of the grant of the patent:
**29.11.2017   Bulletin 2017/48**

(51) Int Cl.:
*A61K 31/404* *(2006.01)*          *A61K 35/60* *(2006.01)*
*A61K 47/30* *(2006.01)*          *A61P 31/12* *(2006.01)*

(21) Application number: **14804573.5**

(22) Date of filing: **19.05.2014**

(86) International application number:
**PCT/RU2014/000360**

(87) International publication number:
**WO 2014/193269 (04.12.2014 Gazette 2014/49)**

(54) **DIINDOLYLMETHANE-BASED MEDICINAL AGENT AND USE THEREOF TO TREAT INFLUENZA AND VIRAL RESPIRATORY INFECTIONS**

DIINDOLYLMETHANBASIERTES ARZNEIMITTEL UND VERWENDUNG DAVON ZUR BEHANDLUNG VON INFLUENZA UND VIRALEN ATEMWEGSINFEKTIONEN

MÉDICAMENT À BASE DE DIINDOLYLMÉTHANE ET SON UTILISATION POUR TRAITER LA GRIPPE ET LES INFECTIONS VIRALES RESPIRATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **28.05.2013   RU 2013124416**

(43) Date of publication of application:
**11.05.2016   Bulletin 2016/19**

(73) Proprietor: **Kiselev, Vsevolod Ivanovich**
**Moscow 117218 (RU)**

(72) Inventor: **Kiselev, Vsevolod Ivanovich**
**Moscow 117218 (RU)**

(74) Representative: **Einsel, Martin**
**Patentanwälte**
**Einsel & Kollegen**
**Jasperallee 1A**
**38102 Braunschweig (DE)**

(56) References cited:
**WO-A1-2012/022101      RU-C1- 2 350 601**
**RU-C1- 2 419 426      RU-C2- 2 318 509**
**US-B1- 6 689 387**

- **AUBORN K J: "THERAPY FOR RECURRENT RESPIRATORY PAPILLOMATOSIS", ANTIVIRAL THERAPY- AN OFFICIAL PUBLICATION OF THE INTERNATIONAL SOCIETY FOR ANTIVIRAL RESEARCH, MTM PUBLICATIONS, LONDON, GB, vol. 7, no. 1, 1 January 2002 (2002-01-01) , pages 1-9, XP008072967, ISSN: 1359-6535**

**Description**

The present invention relates to the field of medicine, more specifically to pharmacology, and can be used to treat influenza and viral respiratory infections.

The growing part of infectious viral diseases in the structure of the population general morbidity, the origination of new hazardous viruses showed with entire evidence that the public health faced the problem of deficiency of antiviral preparations for treating heavy forms of viral infections. For the last years, the development of antiviral preparations has become one of the pressing directions of modern pharmacology. The crisis of these studies appeared with a particular evidence in 2003 when practical health care in many countries met face-to-face an unprecedented mortality of patients with atypical pneumonia caused by the coronavirus SARS. Actually, to treat this heavy and practically fatal infection, the World Health Organization (WHO) recommended only one preparation, Ribavirin having at the same time many problems. As the unique inhibitor of the synthesis of RNA-containing viruses, Ribavirin represents, with no doubt, a high achievement of modern pharmacotherapy, but clinic physicians know well serious side-effects arising in the use of this preparation. The development of preparations against RNA-containing viruses remained outside the attention of big producers and developers of medicinal preparations as well as outside the attention of WHO. Nevertheless, keeping without any reaction the problem of healing SARS, hemorrhagic Ebola, Dengue fevers and influenza cost life to many sick people in various world countries. The real mortality from the "bird" flu A(H5N1) in the South-Eastern countries showed once more the impotence of modern medicine. The A(H1N1)pdm2009 flu pandemic built up in 2009 accentuated the problem of availability and efficiency of medicinal agents to treat this heavy infection. A vast morbidity and a relatively high death rate have increased the demand for national, accessible to population, low-priced and efficient preparations for preventing and treating influenza.

WHO recommended the use of preparations with etiotropic effect to treat and to prevent influenza. At present, two generations of such preparations are used in the world. The first one is represented by the well-known rimantadine and by amantadine, similar to the last and used in Europe and USA. The second generation preparations are represented by selective inhibitors of viral neuraminidase named Relenza (Zanamivir) and Tamiflu (Oseltamivir). Besides them, a series of other preparations is widely used in Russia, such as Arbidol, Cycloferon, Ingavirin, Kagocel, interferon and other preparations related to the class of immunomodulators. But already in 2006 and after the pandemic flu in 2009, in the period of use of the Tamiflu preparation to treat heavy conditions of the "bird" flu in people, the preparation was seriously compromised both as to clinical efficiency and as to a wide range of side-effects, and later as to a fast spread of genetic indices of the virus strains resistance to Tamiflu. Besides, the practice of use of the neuraminidase inhibitors in the treatment of influenza showed that a high efficiency of this group of preparations was limited by their prescribing at an early stage of the infection, the efficiency abruptly going down two days after the disease onset.

Thus, after the analysis of the main preparations for treating influenza, it is worth to note that all of them are not drawback-free, and the main drawbacks consist in the growth of resistance, the need of an early use of preparations, their toxicity and the appearance of side-effects, the non-availability of preparations to treat heavy forms of influenza infections.

Acute respiratory viral infections (ARVI) and influenza considered as socially-significant infections remain practically uncontrollable diseases due to a high variability of the antigen structure, the virulence of circulating viruses and the appearance of new highly pathogenic infectious agents (SARS, "bird" flu A(H5N1) viruses, A(H1N1)pdm2009 flu pandemic virus. A direct danger to child's life is represented by ARVIs that are accompanied by a larynx lesion (adenoviruses, parainfluenza viruses, herpes viruses, respiratory syncytial virus). But no etiotropic agents exist to treat or to prevent many of these infections, since existing etiotropic antiviral agents (Rimantadine, Tamiflu, Ribavirin) are not free of disadvantages the main of which to be cited are growth of resistance, of toxicity, appearance of side-effects, a limited range of action, etc.

What is more, no etiotropic agents exist at all against many respiratory viruses to provide treatment or prevention. It regards such widespread viral infections like respiratory syncytial virus, parainfluenza, adenoviral infection and some others. At the same time, said infections are widespread and often induce heavy diseases both amongst adults and children, which raises a question of the need to develop new preparations to provide an optimal assortment of medicinal agents to treat said infections.

As it has been shown for the last years, many viruses including H1N1 and H5N1 flu viruses meddle significantly in the mechanisms of apoptosis of host cells. At the same time, it is well known that a long secretion of anti-inflammatory cytokines by virus-infected macrophages can in turn induce the T-lymphocyte apoptosis, suppressing by the same the immune response to the virus and induce the apoptosis of surrounding tissue cells, aggravating the injury of the same. Besides, in this case the infected macrophages synthesize an important number of active forms of oxygen injuring the surrounding tissues.

An alternative possibility to influence the viral infection pathogenesis is to inhibit the secretion of anti-inflammatory cytokines which is considered as one of the main factors leading to heavy injuries of organs and tissues in the case of a series of viral infections. In particular, it relates to such serious infections that are induced by the H5N1 flu and atypical pneumonia viruses (Korteweg C., Gu J. (2008). Pathology, molecular biology, and pathogenesis of avian influenza A (H5N1) infection in humans, Am. J. Pathol., 172(5), 1155-1170).

Seemingly, the inhibition of the apoptosis of infected microphages and the hypersecretion of anti-inflammatory cytokines can be considered as ones of the most important pathogenetic mechanisms that allow the development of influenza infection in the organism. Taking into consideration the data on the implication of these mechanisms into the infectious process of influenza, one can admit that the recovery of the macrophage ability to apoptosis and the inhibition of the excessive secretion of anti-inflammatory cytokines should lead to an accelerated elimination of the influenza virus and to a lower degree of tissue injuries.

Literature discloses a lot of arguments to support the fact that 3,3-diindolylmethane (DIM) commits the cells to the apoptosis running with the participation of the mitochondrial mechanism and probably of other mechanisms.

To treat acute infectious diseases, the rapidity for delivering a medicinal agent to the infection focus is particularly important. Such a medicinal form was developed for 3,3-diindolylmethane (RU 2419426 C1, published on the 27.05.2011) and represents DIM dissolved in a mixture of fish oil and of a polysorbate, packed into solid gelatin capsules. The fish oil content level at which one succeeds to provide the DIM dissolving and to increase the bioavailability of the same in the case of oral administration is of 10-20% of the total mass of the capsule content.

The composition of the solution to fill the capsules has been developed with the use of fish oil of the "Omega-3"-type that has improved the substance solvability. The selected "Omega-3" component ratio to the total solution mass of 10 to 20% has provided the stability both of the solution and of the preparations for a selected time period. The preparation composition according to patent RU 2419426 is given in Table 1. The characteristics of the "Omega-3" fish oil are given in Table 2.

**Table 1.**

| Content per capsule | 1 | 2 | 3 |
| --- | --- | --- | --- |
| Diindo lylmethane | 50 mg | 100 mg | 150 mg |
| Omega-3 (Fish oil) | 100 mg | 100 mg | 100 mg |
| *Adjuvant substances* | | | |
| Polysorbate 80 (EP 2007) | 400 mg | 350 mg | 500 mg |
| Mass of the capsule content | 550 mg | 550 mg | 750 mg |

**Table 2.**

| Omega-3 | | |
| --- | --- | --- |
| Description of indices | Method | Requirements |
| Aspect | Organoleptic method | Transparent yellowish liquid with a fish odor |
| Solubility | State Pharmac. XII | Practically insoluble in water |
| Relative density | Areometer | 0.915-0.930 g/cm$^3$ |
| Acid number | Europ. Pharmac. | No more than 2.0 mg KOH/g |
| Peroxide number | Europ. Pharmac. | No more than 5.0 mmol act.acid/kg |
| Tocopherol (Vitamin A) | Europ. Pharmac. | Up to 20 ppm |
| *Omega-3 Total:* | Gas-Liquid Chrom. | 60.0-100.0% |
| *Polyunsaturated fatty acids* | | |
| Timnodonic (eicosopantaenoic) acid (EPA) | Gas-Liquid Chrom. | 33-44% |
| Cervonic (docosohexaenoic) acid (DHA) | Gas-Liquid Chrom. | 22-30% |

The use of the formulation described in the patent RU 2419426 for treating influenza infection showed a side-effect under the form of a heavy form of diarrhea that forced to stop further administering of the preparation. As we assume, this side-effect was due to a high content of fish oil stimulating the intestinal peristalsis. Thus, the formulation described cannot be used for treating any acute influenza infection, especially considering the fact that for obtaining stable clinical effects, high DIM dosages up to 0.5 g and more are necessary.

The aim of the present invention is to broaden the range of agents available for treating acute respiratory diseases and influenza infection by developing an oral medicinal agent based on 3,3-diindolylmethane (DIM) for the treatment of these diseases.

The technical result of the present invention consists in providing a possibility to treat acute respiratory diseases and influenza infection with the use of DIM.

The technical result of the present invention is achieved with a medicinal agent for treating influenza and respiratory viral infections, containing 3,3-diinolylmethane, A-type fish oil and an adjuvant, with the component ratio in mass %:

| | |
|---|---|
| 3,3-diindolylmethane | 9-20 |
| A-type fish oil | 1-10 |
| Adjuvant | balance. |

In this case, the medicinal agent preferably contains polysorbate 80 as an adjuvant.

To solve the mentioned task, first of all, it was necessary to lower the content of fish oil in the diindolylmethane preparation without changing, at the same time, the DIM solubility in the formulation, and to keep a high bioavailability of the active substance.

The technical result is achieved as well by the use of said medicinal agent to treat influenza and respiratory viral infections, preferably by means of administering the same at the ratio of 1 to 10 mg per kg of patient body weight.

The authors have assumed that the present task can be solved with the use of A-type fish oil, characterized by a low content of unsaturated fatty acids (Example 1).

The study of the antiviral activity of different medicinal forms of diindolylmethane was carried out on the basis of an analytical model of the preparation protective properties in laboratory animals (white mice) against an experimental lethal influenza infection. Predetermined conditions of experimental studies enable one to perform a severe selection of preparations providing for a high percentage of animal survival after infection with multiple lethal virus dosages. Actually, under natural conditions, a man gets never infected with an absolutely lethal dosage of a virus. During the incubation period and the 1st phase of the process development, the virus replicates in the human organism, and the main condition of a complicated infection, dangerous for the life is to inhibit protective mechanisms by the virus. In an animal model, after infection with a lethal dosage of virus, these mechanisms are suppressed immediately, which enables one to test preparations that affect the main processes of the virus reproduction and protection.

**Materials and methods.**

*Viruses.* The A/Aichi/2/68 (H3N2) influenza virus was obtained from the virus collection of the Scientific Research Institute for Influenza of the Russian Academy of Medical Sciences and was passaged in the allantoic cavity of 10-12-day hen's embryos for 48 hours at 36°C.

*Animals.* White mongrel mice (females) with the mass of 14-16 g were obtained from the "Rappolovo" nursery (Leningrad region) and attended with a standard ration under the controlled conditions of the vivarium at the Scientific Research Institute for Influenza of the Russian Academy of Medical Sciences. The animal selection to experimental groups was carried out by the random selection method. Before tests, the animals were under observation for 2 weeks.

*Experimental influenza infection.* Before the experiment, 5 mice were infected with a virus-containing allantoic liquid. On the third day after infection, their lungs were isolated, homogenized in a 10-fold volume of a sterile physiologic solution, and then the infectious activity of the virus in a homogenized solution was determined in a special experiment via lethality titration on animals. The virus titer was calculated by the Reed-Muench method (Am.J.Hyg.,1938, 27:493-497).

The preparations under study were administered orally to animals on the basis of the concentration of 150 mg/kg of weight with the 0.2 ml volume. The preparations were administered according to the following treatment-and-prophylactic scheme: 24 hours and 1 hour before infection and 24, 48 and 72 hours after infection. As placebo, phosphate buffer was administered to a control group of animals. As a reference preparation, Rimantadine was injected to animals intraperitoneally in the dosage of 0.2 ml according to the same diagram that for the investigated preparations. As a negative control, intact animals were used that were attended under the same conditions that the experimental animals.

The virus was administered intranasally under a slight ether anesthesia in the dosage of 1 and 10 $LD_{50}$. 13 mice were taken to any observation group. On the 3rd day after infection, 3 animals of each group were sacrificed, autopsied and their lungs were separated to isolate the virus. The lungs were kept at -20°C until setting up respective experiments.

Remaining animals were observed for 14 days, i.e. the period within which the mortality of animals was recorded.

The deaths of animals were recorded daily in the control and experimental groups. On the basis of the data obtained, the death rate was calculated for each group (M being the ratio of the number of dead animals in 14 days to the total number of infected animals in the group), the protection index (IP being the ratio of the difference between the mortality percentage in the control and in the experimental groups to the mortality percentage in the control group) and the average life duration of animals (DL) on the basis of 14 days of observation in accordance with the following formulas:

$$DL = (\Sigma \, N \, D)/Nt,$$

where

N is the number of animals that survived D days,

Nt is the total number of animals in a group;

**M=M/Nt,** where M is the number of animals in a group that died within 14 days after infection;

**IP=((Mc-Me)/Mc)x100%**, where Mc and Me represent mortality in percentages in the control and experimental groups, respectively.

The animals that survived to the 15th day after infection were autopsied and their lungs were studied macroscopically. Visual evaluation was carried out of the surface of chronic foci of postinfluenzal pneumonia remaining in the lung tissue of mice after the acute stage of the influenza infection within the entire life of the animal. The surface of the lesion foci was expressed in percentage to the entire lung surface.

*Virus titration in lung tissues.* To determine the infectious titer of the influenza virus in the lung tissue of animals, the mice lungs extracted on the 3rd days after infection were homogenized in a 10-fold volume of a sterile physiological phosphate buffer and the homogenized solutions were used to get ready a series of 10-fold dissolutions with the same buffer. To titer the virus, use was made of fragments of survived chorioallantoic membranes (CAM) from hen's embryons.

The studies were carried out according to a modified according to the modified Fazekas de St.Growts method [Hyng J., 1958, p. 151]. CAM fragments were prepared from chorioallantoic membranes of 10-12-day's hen's embryos after their previous sterilizing with ethanol and after elimination of the embryon together with the vitellicle. Fragments of CAM with the size of 20 x 25 mm were placed into wells of polystyrene panels each containing 0.2 ml of cell maintenance medium and 0.2 ml of lung homogenized solution with respective dilution. The plates were covered with glass, kept for 48 hours at the temperature of 37°C, the CAM fragments were eliminated and 0.4 ml of 1% suspension of hen's erythrocytes in the physiological solution were added.

The level of the virus reproduction in the panel wells was evaluated after the hemagglutination reaction (HAR) of erythrocytes. For a virus titer, the value was adopted opposite to the decimal logarithm of the maximal dilution of the virus able to induce a positive hemagglutination reaction, this value being expressed in logarithms of the minimal 50% dosage of the virus ($lg \, ID_{50}$).

The invention is illustrated by the following Figures.

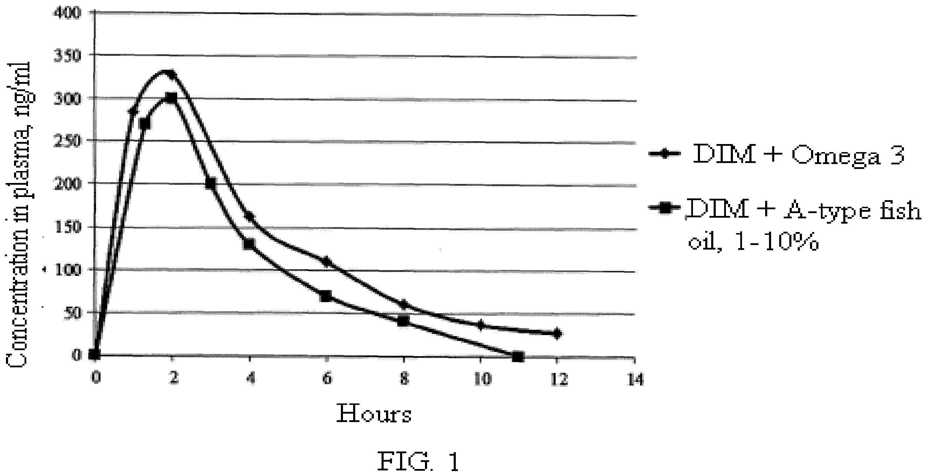

Fig. 1 shows a diagram of the time-dependence of diindolylmethane concentration in the plasma of volunteers after administration of 100 mg of the agent according to the present invention.

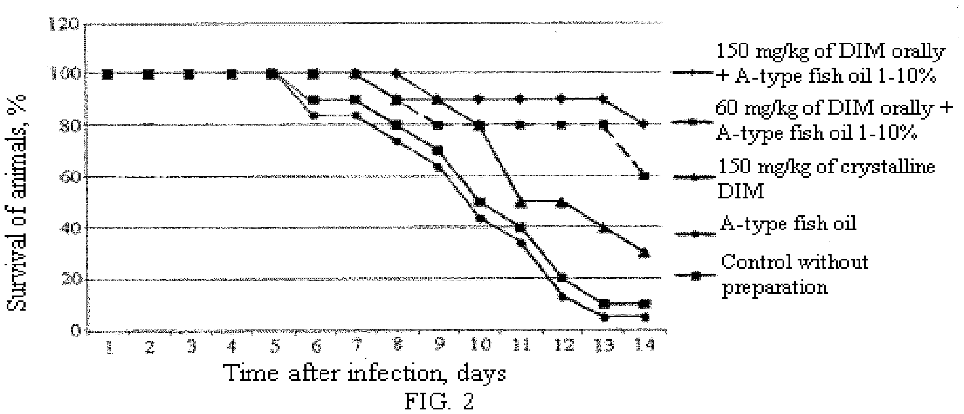

Fig. 2 shows diagrams of the white mice survival dependence in time after their infection with lethal influenza pneumonia at various administrations as follows: (1) DIM agent of the present invention of 150 mg/kg orally +1% of A-type fish oil; (2) DIM agent of the present invention of 600 mg/kg orally +10% of A-type fish oil; (3) only 150 mg/kg of crystalline DIM; (4) only A-type fish oil; (5) a control without any medicinal preparation.

The invention is illustrated by the following examples.

*Example 1.*

**Composition of fish oil used to prepare a medicinal formulation.**

Table 3 shows the specification of the fish oil used to obtain a medicinal formulation of the present invention.

**Table 3.**

| | A-type Fish oil (EP) | |
|---|---|---|
| Description of indices | Method | Requirements |
| Aspect | Organoleptic method | Transparent yellowish liquid with a fish odor |
| Solubility | State Pharmac. XII | Practically insoluble in water |
| Relative density | Areometer | 0.917-0.930 g/cm$^3$ |
| Acid number | Europ. Pharmac. 2.5.1 | No more than 2.0 mg KOH/g |
| Peroxyde number | Europ. Pharmac. 01/2008: 0428 | No more than 10.0 mmol act.ac./kg |
| Vitamin A | Europ. Pharmac. | 600-2500 IU |
| Vitamin D$_3$ | Europ. Pharmac. | 60-250 IU |
| *Omega-3 Total:* | Gas-Liquid Chrom. | 60.0-100.0% |
| *Composition of fatty acids:* | | |
| Myristic acid | Gas-Liquid Chrom. | 2.0-6.0% |
| Palmitic acid | Gas-Liquid Chrom. | 7.0-14.0% |
| Palmitoleic acid | Gas-Liquid Chrom. | 4.5-11.5% |
| Oleic acid | Gas-Liquid Chrom. | 12.0-21.0% |
| *Polyunsaturated fatty acids:* | | |
| Linoleic acid | Gas-Liquid Chrom. | 1.5-3.0% |
| $\alpha$-Linoleic acid | Gas-Liquid Chrom. | 0-2.0% |
| Timnodonic (eicosopantaenoic) acid (EPA) | Gas-Liquid Chrom. | 7.0-16.0% |
| Cervonic (docosohexaenoic) acid (DHA) | Gas-Liquid Chrom. | 6.0-18.0% |

### *Example 2.*

**Formulation of the preparation.**

Table 4 shows the compositions of the preparation per capsule.

**Table 4**

| Content per capsule | | |
|---|---|---|
| Diindo lylmethane | 50 mg | 100 mg |
| A-type Fish oil | 10-55 mg | 10-55 mg |
| *Adjuvant substances* | | |
| Polysorbate 80 (EP 2007) | up to 550 mg | up to 550 mg |
| Mass of the capsule content | 550 mg | 550 mg |

### *Example 3.*

**Concentration of diindolylmethane in the volunteers' plasma after administration of 100 mg of preparation.**

In all the clinical studies, pharmacokinetics comparison was made between the DIM preparation of the invention on the basis of A-type fish oil with the concentration 1-10% of preparation, the DIM preparation on the basis of Omega 3 and the crystalline DIM in the same dosages. It was established for all the studied groups of volunteers that both preparations have a comparable bioavailability. The maximum DIM concentration in blood was observed two hours after the administration of preparations and achieved approximately 300 ng/ml. The administration of crystalline DIM with the same dosage did not enable one to discover it in this range of concentrations in the patients' plasma **(Fig. 1).**

### *Example 4.*

**Influence of chemical preparations on the content of infectious virus in the lung tissue of white mice on the 3rd day after infection, given in Table 5.**

**Table 5.**

| Preparation | Infectious titer of the influenza virus (lg $EID_{50}$) in lungs at the following dosage of virus, lg $LD_{50}$ | | | |
|---|---|---|---|---|
| | 1 | | 2 | |
| 150 mg/kg of DIM orally + A-type fish oil 1-10% | 4.0 3.0 3.0 | **3.3±0.33** | 4.0 3.5 4.4 | **4.0±0.29** |
| 60 mg/kg of DIM orally + A-type fish oil 1-10% | 3.5 3.0 3.5 | **3.3±0.17** | 4.5 4.5 4.0 | **4.3±0.17** |
| 60 mg/kg of crystalline DIM orally | 4.0 3.5 4.0 | **3.8±0.17** | 4.0 4.5 5.0 | **4.5±0.29** |
| 50 mg/kg of rimantadine orally | 2.0 3.0 2.5 | **2.8±0.17** | 2.5 3.0 2.5 | **2.7±0.17** |
| Control without preparations | 4.0 4.0 4.0 | **4.0±0.00** | 4.5 5.0 5.0 | **4.8±0.17** |
| Intact animals | 0.0 0.0 0.0 | | **0.0±0.00** | |
| * average values in each group of animals are marked out in thick prints. | | | | |

The experiment did not show toxic effects of the preparations in used concentrations as dosage-dependent unspecific mortality at early periods of the same (1-2nd days).

As shown by the results provided, the infection of animals with the influenza virus caused a replication of the infectious agent in lungs independently of the infectious dosage until the level 4-5 lg $EID_{50}$/0.2 ml. The use of rimantadine with the dosage of 50 mg/kg of weight of animals reduces this index by a factor of 1 to 3, depending on the virus dosage used.

The use of the preparation on the basis of DIM and of A-type fish oil at a concentration of 1-10% did not significantly affect the virus infectious titers in lungs.

The experiment did not show unspecific mortality in the control group of intact animals. The disease clinical signs were typical for an influenza infection and comprised difficult breathing, ataxia, tremor as well as a lowered consumption of feed and water.

*Example 5.*

**Mortality in the course of a pathological process at experimental lethal influenza infection in white mice caused by the A/Aichi/2/68 (H3N2) influenza virus under the conditions of use of the chemical preparations, given in Table 6.**

**Table 6.**

| Preparation | LD$_{50}$ virus dosage | Mortality of animals after infection (days) | | | | | | | | | | | | | Average life (days) | Protection index, % |
|---|---|---|---|---|---|---|---|---|---|---|---|---|---|---|---|---|
| | | 1 | 2 | 5 | 6 | 7 | 8 | 9 | 10 | 11 | 12 | 13 | 14 | 15* | | |
| 150 mg/kg of DIM orally + A-type fish oil 1-10% | 1 | | | | | | | | | | | | | 0/10 | 15.0 | 100 |
| | 10 | | | | | | 1 | | | | | | 1 | 2/10 | 14.2 | 78 |
| 60 mg/kg of DIM orally + A-type fish oil 1-10% | 1 | | | | | | | | | | | | | 0/10 | 15 | 100 |
| | 10 | | | | | | 1 | 1 | | | | | 2 | 4/10 | 13.5 | 56 |
| Rimantadine | 1 | | | | | | | 1 | | | | | | 1/10 | 14.4 | 80 |
| | 10 | | | | | | 1 | | | | 1 | | | 2/10 | 13.9 | 78 |
| Control without preparations | 1 | | | | | 1 | | 2 | 1 | 1 | | | | 5/10 | 12.1 | --- |
| | 10 | | | | 1 | | 1 | 1 | 2 | 1 | 2 | 1 | | 9/10 | 10.9 | --- |

* - total mortality (number of dead/infected)

As it results from the data given in Table 6, the influenza virus caused lethal infection in white mice, accompanied by the death of animals starting from the 6-9th days after infection, depending on the virus dosage. Such an index like the animal life duration was related to the used virus dosage in inverse dependence. Rimantadine used in the experiment as a reference preparation showed in this infection a significant protective effect, which was manifested by a lower mortality in experimental groups compared to the control (protection index of 78-80%) and by a longer life (from 2.7 to 3.0 days depending on the virus dosage). Thus, the data obtained are in agreement with earlier obtained results of experiments in vitro, which indicates the sensitivity of the used virus strain to rimantadine.

The analysis of the indices dynamics of a pathological process under the conditions of use of chemical preparations showed that they demonstrate as well the ability to increase the period of life of animals and to lower the specific mortality in the experimental groups. In particular, the maximal dosage of the preparation developed, in the case of oral administration, leads to 100% protection of animals against lethal influenza pneumonia. In this case, as to the indices combination, DIM resulted comparable in efficiency with rimantadine, in spite of the absence of direct antiviral activity in the same.

### *Example 6.*

**Protective effect of the designed preparations in the case of lethal influenza pneumonia in white mice. Viral dosage 10 $LD_{50}$.**

The analysis of the indices dynamics for a pathological process under the conditions of use of the designed preparation showed that it demonstrates as well the ability to increase the period of life of animals and to lower the specific mortality in the groups. In particular, the oral administering of the maximal dosage of the designed preparation leads to the 100% protection of the animals against lethal influenza pneumonia.

**Fig. 2** illustrates the protective effect of the preparations with the maximal virus dosage used (10 $LD_{50}$). It is to note that DIM in crystalline form had no practical influence on the survival of infected animals, which is obviously due to a low bioavailability. The results of survival of animals that were fed only with fish oil were comparable to the control group that had no treatment at all.

### *Example 7.*

**The results of evaluation of the foci surface of lung chronic lesion in the case of use of chemical preparations are given in Table 7.**

**Table 7**

| Preparation | Surface of chronic pneumonia foci (% of total surface of the lung surface) at the viral dosage $LD_{50}$ | |
|---|---|---|
| | Viral dosage -1 $LD_{50}$ | Viral dosage -10 $LD_{50}$ |
| 150 mg/kg of DIM orally + A-type fish oil 1-10% | $26\pm8.2$ | $35\pm11.4$ |
| 60 mg/kg of DIM orally + A-type fish oil 1-10% | $4\pm3.2$ | $50\pm2.8$ |
| Rimantadine | $31\pm11.8$ | $27\pm6.8$ |
| Control without preparations | $39\pm8.2$ | $72\pm9.0$ |

As one can see in Table 7, the influenza infection lead to the appearance of stable foci of chronic inflammation in the lungs of animals, the surface of said foci depended directly from the used viral dosage in animals survived on the 15th days of the experiment. The use of rimantadine reduced this index for each infecting dosage, and it was expressed with most certainty at the viral dosage of 10 $LD_{50}$. The use of the designed preparation had a significant effect on this index as well.

*Example 8.*

**Study of the designed preparation efficiency in the treatment of respiratory viral infections in humans.**

The study included patients that satisfied the following criteria:

Inclusion criteria:

- A middle-severity ARVI diagnosis confirmed by a laboratory, induced by viruses of influenza, parainfluenza, adenovirus, respiratory syncytial virus;

- the presence of general infectious intoxication symptoms;

- the presence of symptoms of the upper respiratory tract lesion (rhinitis, pharyngitis, laryngitis, tracheitis);

- the sickness duration does not exceed 48 hours;

- the patient's age is of 18 to 50 years old;

- the absence of complications after ARVI;

Exclusion criteria:

- increased sensitivity, according to the patient's history, to preparations used as standard therapy or to components of the Infemin preparation;

- positive data of the blood count for HIV, syphilis, hepatitis B and C;

- pregnancy or breast feeding;

- now existing or past psychic diseases, now existing alcohol or narcotic dependence;

- autoimmune diseases;

- heavy bodily diseases (chronic diseases of the cardiovascular system, of the neuroendocrinal system, of the liver, of the kidneys);

- the need for a hospital cure of ARVI;

- participation in a clinical study within the last 30 days;

- refusal to use barrier contraception methods during the study;

- intake of antiviral and/or immunomodulating preparations from the moment of the ARVI sickness starting.

It was carried out an open, randomized comparative clinical test in two parallel groups. The test provided for 3 periods: screening test for at most 1 day, treatment period for 6 days, period of posterior observation for 6 days. The patients were observed as ambulatory.

Altogether, 56 patients satisfying the inclusion/exclusion criteria were randomly distributed in 2 groups of 28 patients each. The investigated preparation was prescribed to the first group patients at a dosage of 400 to 800 mg/day (2-4 capsules of 100 mg diindolylmethane dosage twice a day) for 6 days; besides, they received a standard symptomatic treatment. The second group patients received only the standard symptomatic treatment. During the test, the evaluation of the patient condition was performed 5 times for the period of 12 days.

The average length of the sickness in the control group was of $7.0 \pm 1.5$ days, and in the experimental group, it was 5.5±1.5 days. The difference is statistically significant (p=0.0004, Student's criterion). Furthermore, At the end of the therapy course (on the 6th day of treatment), in 24 patients of the experimental group, an increase of the level of the gamma-interferon production induced by lymphocytes was observed whereas it was only in 9 patients en the control group (p=0.0002, criterion $\chi^2$). The number of complications was lower as well on the background of the preparation receiving: in 2 patients, whereas in the control group, it was in 8 people (p=0.0403, criterion $\chi^2$). No statistically significant differences were found as to the preparation efficiency in the subgroups with the infection induced by different viruses, which demonstrates the universality of the curative effect at the considered group of diseases.

**As a result of the performed studies, on can conclude the following:**

1. A test of protective properties of the DIM preparations on the basis of A-type fish oil at the concentration of 1 to 10% to treat and to prevent viral infections in laboratory animals (white mice) in the case of an experimental influenza infection.

2. The DIM preparation on the basis of A-type fish oil at the concentration of 1 to 10%, used orally according to a treatment-and-prophylactic chart did not influence the influenza virus replication in lungs of infected animals.

3. It was shown that the use of DIM preparations on the basis of A-type fish oil at the concentration of 1 to 10% prevents the development of chronic consequences of the influenza infection, while reducing the area of the chronic postinfluenzal pneumonia foci in animals.

4. A high protective activity of the DIM preparations on the basis of A-type fish oil at the concentration of 1 to 10% was shown in the cases of oral administration. This activity is manifested by a lower mortality of mice in the experimental groups compared to the control group (protection index depending on the preparation dosage is of 80 to 100% and of 22 to 78% at the infectious dosage of 1 and of 10 $LD_{50}$, respectively), as well as by a higher average life period (increase of 2.5 to 2.9 and of 1.4 to 3.3 days at virus dosages of 1 and 10 $LD_{50}$, respectively). Thus, the activity of this preparation in oral administration becomes comparable or superior to the activity of rimantadine, as a comparison preparation.

5. It was shown that the use of DIM preparations on the basis of A-type fish oil at the concentration of 1 to 10% prevents the development of chronic consequences of an influenza infection, while reducing significantly the area of the chronic postinfluenzal pneumonia foci in animals.

**Claims**

1. A medical agent for use in treating influenza and viral respiratory infections, containing 3,3-diindolylmethane, A-type fish oil and an adjuvant, with the following ingredient ratio, in % by mass:

| | |
|---|---|
| 3,3-diindolylmethan | 9-20 |
| A-type fish oil | 1-10 |
| adjuvant | balance. |

2. The medical agent for use according to claim 1, **characterized in that** it contains polysorbate 80 as an adjuvant.

3. The medical agent for use according to claim 1 where the medicinal agent is administered in an amount of 1 to 10 mg of 3,3-diindolylmethane per kilogram of patient body weight.

**Patentansprüche**

1. Ein medizinischer Wirkstoff zur Verwendung bei der Behandlung von Influenza und viralen Atemwegsinfektionen enthaltend 3,3-Diindolylmethan, A-Typ-Fischöl und einen Hilfsstoff,
wobei das Verhältnis der Bestandteile in Masseprozent wie folgt ist:

| | |
|---|---|
| 3,3-Diindolylmethan | 9-20 |
| A-Typ-Fischöl | 1-10 |
| Hilfsstoff | Rest |

**2.** Der medizinische Wirkstoff für eine Verwendung nach Anspruch 1,
**dadurch gekennzeichnet,**
**dass** er Polysorbat 80 als Hilfsstoff enthält.

**3.** Der medizinische Wirkstoff für eine Verwendung nach Anspruch 1, wobei der medizinische Wirkstoff in einer Menge von 1 bis 10 mg 3,3-Diindolylmethan pro Kilogramm Körpergewicht des Patienten verabreicht wird.

**Revendications**

**1.** Agent médical pour une utilisation dans le traitement de la grippe et des affections respiratoires virales, contenant du 3,3-diindolylméthane, de l'huile de poisson de type A et un adjuvant, avec le rapport des composants suivants en % en masse :

| | |
|---|---|
| 3,3-diindolylméthane | 9 à 20 |
| huile de poisson de type A | 1 à 10 |
| adjuvant | complément à 100 %. |

**2.** Agent médical pour une utilisation selon la revendication 1, **caractérisé en ce qu'**il contient du polysorbate 80 en tant qu'adjuvant.

**3.** Agent médical pour une utilisation selon la revendication 1 où l'agent médicinal est administré dans une quantité de 1 à 10 mg de 3, 3-diindolylméthane par kg de poids corporel du patient.

FIG. 1

EP 3 017 816 B1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 2419426 C1

- RU 2419426

### Non-patent literature cited in the description

- **KORTEWEG C. ; GU J.** Pathology, molecular biology, and pathogenesis of avian influenza A (H5N1) infection in humans. *Am. J. Pathol.,* 2008, vol. 172 (5), 1155-1170

- *Am.J.Hyg.,* 1938, vol. 27, 493-497